# EUROPÄISCHE PATENTSCHRIFT

(11) **EP 1 003 463 B1**
(45) Veröffentlichungstag und Bekanntmachung des Hinweises auf die Patenterteilung: **12.06.2002**
(21) Anmeldenummer: 98947329.3
(22) Anmeldetag: 21.07.1998
(51) Int. Cl.: A61K 7/00

(54) **KOSMETISCHE ZUSAMMENSETZUNGEN MIT AGGLOMERIERTEN SUBSTRATEN**
COSMETIC COMPOSITIONS WITH AGGLOMERATED SUBSTRATES
COMPOSITIONS COSMETIQUES A SUBSTRATS AGGLOMERES

(30) Priorität: 01.08.1997 DE 19734547
(43) Veröffentlichungstag der Anmeldung: 31.05.2000
(73) Patentinhaber: LANCASTER GROUP GmbH, 55116 Mainz (DE)
(72) Erfinder: GOLZ-BERNER, Karin, MC-98000 Monaco (MC); ZASTROW, Leonhard, MC-98000 Monaco (MC)
(74) Vertreter: Walter, Wolf-Jürgen
(86) Internationale Anmeldenummer: DE9802087
(87) Internationale Veröffentlichungsnummer: WO9906012

(56) Entgegenhaltungen:
- EP-A- 0 406 657
- EP-A- 0 704 502
- WO-A-96/17588
- US-A- 5 082 660
- US-A- 5 234 682

## Beschreibung

Die Erfindung betrifft kosmetische Zusammensetzungen, die Agglomerate bestimmter Teilchengrößen enthält.

Es ist bekannt, daß anorganische Teilchen, wie z. B. Oxide wie Eisenoxide, Titaniumdioxid, Zinkoxid usw. dazu neigen, in kosmetischen Emulsion zu Agglomeraten unterschiedlicher Größe zusammenzutreten, wodurch der Auftrag derartiger Emulsionen auf die Haut unangenehm sein kann, wenn große
Agglomerate erst verrieben werden müssen. Das Problem wächst in dem Maße, wie sich die Teilchengrößen der eingesetzten Produkte verringern. Daher muß mit anderen Zusatzstoffen oftmals der Agglomeratbildung entgegengewirkt werden.

Andererseits sind aus der EP-B-406657 plättchenförmige Substrate bekannt, die einen Gehalt von wenigstens 0,5 Gew-% sphärische Teilchen mit im Vergleich zu den plättchenförmigen Substraten kleinem Durchmesser von etwa 0,05 bis 50 µm aufweisen. Als besonderer Vorteil der Erfindung wird ausgewiesen, daß durch den Zusatz der sphärischen Teilchen aus z. B. SiO₂, TiO₂ und ZrO₂ eine Deagglomerierung der plättchenförmigen Substrate auftritt.

Weiterhin wurde in der WO 96/17588 gefunden, daß durch Zusatz von SiO₂ zu Kaolin, der ebenfalls plättchenförmige Substrate ausbildet, eine Erhöhung des Kaolingehaltes in kosmetischen Zusammensetzungen ermöglicht wird.

Die EP-A-704502 beschreibt die Verbesserung der Schutzwirkung von ZnO durch Dispersion in einer Alkalimetallsilicatlösung, die durch Zusatz von CO₂ geliert wird, wobei ZnO-haltiges SiO₂ erhalten wird.

Der Erfindung liegt die Aufgabe zugrunde, kosmetischen Zusammensetzungen mit sehr guten Verteilungseigenschaften auf der Haut zu entwickeln, ohne daß die Agglomerierung anorganischer Partikel, wie oxide, störend wirkt.

Überraschenderweise wurde gefunden, daß der Zusatz von sphärischen, unporösen SiO₂-Teilchen bestimmter Teilchengröße zu anderen im wesentlichen sphärischen Teilchen keine De-agglomerierung bewirkt, sondern zu definierten Agglomeraten führt, deren Teilchengröße für kosmetische Zubereitungen besonders vorteilhaft ist, da diese Zusammensetzungen eine sehr homogene Verteilung auf der Haut ermöglichen, eine sehr gute Reflexion der UV-Strahlung zeigen und in kosmetischen Sonnenschutzzubereitungen mit TiO₂ und/oder ZnO mindestens gleiche Lichtschutzfaktoren wie bekannte Sonnenschutzzubereitungen, jedoch eine höhere Stabilität des Lichtschutzfaktors (SPF) erreichen.

Erfindungsgemäß bereitgestellt werden daher kosmetische Zusammensetzungen mit agglomerierten Substraten, die einen Gehalt an sphärischen unporösen SiO₂-Teilchen aufweisen, wobei die SiO₂-Teilchen eine Teilchengröße im Bereich von 0,05 µm bis 1,5 µm haben, und neben den SiO₂-Teilchen andere anorganische teilchenförmige Stoffe mit sphärischer Struktur vorliegen, wobei die sphärischen SiO₂-Teilchen mit den anderen anorganischen Stoffen definierte Agglomerate mit einer Teilchengröße im Bereich von 0,06 µm bis 5 µm bilden. Dabei liegt der Anteil der Agglomerate in der kosmetischen Zusammensetzung im Bereich von 0,1 bis 30 Gew-%.

Die agglomerierten Substrate sind weiterhin gekennzeichnet dadurch, daß sie hergestellt sind durch Vermischen der sphärischen, unporösen SiO₂-Teilchen und der anderen anorganischen teilchenförmigen Stoffe mit sphärischer Struktur, die trocken oder als ölige Dispersion mit einer Viskosität von 13500 bis 200000 mPa·s (cp) vorliegen, einzeln oder im Gemisch unter Einhaltung eines pH-Wertes von 5,5 bis 7 in der Weise, daß ein Teil des Gesamtwassers bis zum Erreichen einer pastenförmigen Konsistenz hinzugegeben und 20 bis 100 Minuten gerührt wird, dann das restliche Wasser unter Beibehaltung des pH-Wertes hinzugegeben und bei 3000 bis 5000 U/Min homogenisiert wird, wobei die Temperatur während der Mischvorgänge im Bereich von 35 bis 48 °C liegt. Die erhaltene Dispersion wird dann in üblicher Weise mit einem Anteil der Agglomerate an der kosmetischen Zusammensetzung im Bereich von 0,1 bis 30 Gew-% in die kosmetische Zusammensetzung eingearbeitet.

Besonders vorteilhaft einzusetzende SiO₂-Teilchen sind hochmonodisperse, unporöse, sphärische SiO₂-Teilchen gemäß DE 3616133, die durch hydrolytische Polykondensation von Tetraalk-oxysilan in wäßrig-alkoholisch-ammoniakalischen Medium erzeugt werden, wobei ein Sol von Primärteilchen erzeugt wird und anschließend durch ein kontinuierliches, nach Maßgabe des Abreagierens kontrolliertes Zudosieren von Tetraalkoxysilan die erhaltenen SiO₂-Teilchen auf die gewünschte Teilchengröße von etwa 0,05 bis 10 µm bringt.

Es können jedoch auch nach anderen Verfahren erzeugte SiO₂-Teilchen eingesetzt werden, sofern sie unporös und sphärisch sind und die entsprechende Teilchengröße aufweisen. Bei gewünschtem Einsatz von TiO₂ in der Zusammensetzung, können auch solche Partikel verwendet werden, die zu etwa 80 % aus SiO₂ und 20 % aus TiO₂ bestehen.

Die in der erfindungsgemäßen kosmetischen Zusammensetzung verwendeten anderen anorganischen teilchenförmigen Stoffe mit sphärischer Struktur sind zumeist Oxide, wie Eisenoxide, z. B. Fe₂O₃, Fe₃O₄, FeO oder Mischoxide; Titaniumdioxid; Zinkoxid; Zirkoniumdioxid; Pigmente; oder hartmagnetische Einbereichsteilchen (Einkristalle) aus Bariumhexaferrit oder Strontiumhexaferrit mit großer Koerzitivfeldstärke von 4000 bis 5000 Oersted gemäß WO 95/03061.

Weitere Pigmente können z.B. sein Glimmer, Kaolin, Talkum, Nylonkügelchen, Keramikkügelchen, expandierte und nichtexpandierte synthetische Polymerpulver, pulverförmige natürliche organische Verbindungen wie gemahlene Festalgen, verkapselte und unverkapselte Getreidestärken sowie Anlagerungsprodukte mit organischen Farbstoffen.

Der Begriff "anorganische sphärische Teilchen" bedeutet im Zusammenhang mit der vorliegenden Erfindung, daß die Teilchen eine runde kugelförmige bis höchstens ellipsoide Form haben.

Der Anteil der Agglomerate an der Gesamtzusammensetzung kann im Bereich von 0,1 bis 30 Gew-% liegen, vorzugsweise im Bereich von 0,5 bis 25 Gew-%.

Je nachdem, welche Wirkungen durch die anderen anorganischen teilchenförmigen Stoffe ausgeübt werden sollen, kann ihr Verhältnis untereinander sehr unterschiedlich sein. Wenn z. B. bei Sonnenschutzzubereitungen eine Wirkung gegen UVA-Strahlung im Vordergrund steht, kann das Verhältnis TiO₂:ZnO im Bereich von 1:1 bis 1:10 liegen. steht die Wirkung gegen UVB-Strahlung im Vordergrund, kann das Verhältnis TiO₂:ZnO im Bereich von 100:1 bis 20:1 liegen.

Das Verhältnis TiO₂:SiO₂ oder ZnO:SiO₂ kann im allgemeinen zwischen 100:1 bis 1:1 liegen, vorzugsweise 20:1 bis 2:1.

Bei einer Kombination von TiO₂/ZnO/SiO₂ kann das Verhältnis untereinander im Bereich von TiO₂:ZnO:SiO₂ = 1:100:1 bis 100:1:1 liegen.

Auch das Verhältnis anderer anorganischer teilchenförmiger Stoffe zu SiO₂ kann zwischen 100:1 bis 1:1 liegen.

Neben den erfindungsgemäßen definierten Agglomeraten können übliche kosmetische Hilfs- oder Wirkstoffe in der Zusammensetzung in einem Anteil von 99,9 bis 70 Gew-% vorhanden sein.

Zu den kosmetischen Wirkstoffen gehören z. B. Emulgatoren, anorganische und organische Lichtschutzmittel, Radikalfänger, Feuchthaltemittel, Vitamine, Enzyme, pflanzliche Wirkstoffe, Polymere, Melanin, Antioxidationsmittel, entzündungswidrige natürliche Wirkstoffe, mit Sauerstoff beladene asymmetrische lamellare Aggregate gemäß WO 94/00109; Aufschlußprodukte von Hefen oder pflanzlichen Stoffen, hergestellt durch ein schonendes Ultraschall-Aufschlußverfahren gemäß WO 94/13783, Kaolin sowie mit SiO₂ modifiziertes Kaolin gemäß WO94/17588.

Zu den kosmetischen Hilfsstoffen gehören Wasser, verschiedene pflanzliche öle, Mineralöl, Silikonöl, Gelbildner, Farbstoffe, Konservierungsmittel, Parfüm, ein- und mehrwertige Alkohole, Schutzmittel, pH-Regulatoren, Wachse usw.

Bei erfindungsgemäßen kosmetischen Zubereitungen mit TiO₂ und/oder ZnO werden Lichtschutzfaktoren erhalten, die bei gleichem Wirkstoffgehalt wenigstens gleich denen sind, wie sie aus üblichen Sonnenschutzzubereitungen bekannt sind, z. B. aus der EP-B-433086. Darin ist eine synergistische TiO₂/ZnO-Kombination beschrieben, die TiO₂ mit kleiner als 35 nm und ZnO kleiner als 50 nm beansprucht, und bei der TiO₂ mit 2 bis 25 Gew-% enthalten sein kann. Der Lichtschutzfaktor (SPF) nach der Methode von Cole und VanFossen soll bei 24,5 % TiO₂ und 10 % ZnO den Wert 32 haben und die Zusammensetzung ein ästhetisches Aussehen haben.

Die erfindungsgemäßen Sonnenschutzzubereitungen haben jedoch bei wenigstens etwa gleichem Lichtschutzfaktor eine höhere Stabilität des Lichtschutzfaktors, d. h. die Wirkung hält länger an. Dies wurde durch mehrere Untersuchungen eindeutig nachgewiesen. Gegenüber anderen bekannten Zusammensetzungen wird bei der erfindungsgemäßen Zusammensetzung auch der Weißeln-Effekt reduziert.

Die kosmetische Zusammensetzung mit agglomerierten Substraten kann als O/W-Emulsion, W/O-Emulsion, Mehrfachemulsion (O/W/O oder W/O/W) oder als Gel vorliegen. Es können solche Anwendungsformen hergestellt werden, wie Cremes, Lotionen, Gele, Make up's, Lippenstifte, Puder, Masken, Sprays, Sonnenschutzzubereitungen verschiedenster Art, Sonnenvorbräuner, wachsartige Produkte usw.

Die Erfindung betrifft auch die Herstellung der kosmetischen Zusammensetzung mit agglomerierten Substraten. Die Herstellung erfolgt in der Weise, daß sphärische, unporöse SiO₂-Teilchen und andere anorganische teilchenförmige Stoffe mit sphärischer Struktur, die trocken oder als ölige Dispersion mit einer Viskosität von 13500 bis 200000 mPa·s (cp) vorliegen, einzeln oder im Gemisch unter Einhaltung eines pH-Wertes von 5,5 bis 7 in der Weise vermischt werden, daß ein Teil des Gesamtwassers bis zum Erreichen einer pastenförmigen Konsistenz hinzugegeben und 20 bis 100 Minuten gerührt wird, dann das restliche Wasser unter Beibehaltung des pH-Wertes hinzugegeben und bei 3000 bis 5000 U/Min für 20 bis 60 min homogenisiert wird, wobei die Temperatur während der Mischvorgänge im Bereich von 35 bis 48 °C liegt. Dabei bildet das sphärische SiO₂ mit den anderen anorganischen Stoffen definierte Agglomerate mit einer Teilchengröße im Bereich von 0,06 µm bis 5 µm. Die erhaltene Dispersion wird dann in üblicher Weise mit einem Anteil der Agglomerate an der kosmetischen Zusammensetzung im Bereich von 0,1 bis 30 Gew-% in die kosmetische Zusammensetzung eingearbeitet. Wenn die anderen anorganischen Stoffe in der üblichen Pulverform vorliegen, ist es vorteilhaft, das Vermischen mit dem SiO₂-Teilchen bei 35 bis 45° C und weniger als 200 U/Min für 3 bis 20 Minuten durchzuführen.

Falls bereits Dispersionen der anderen anorganischen Stoffe vorliegen, so sind diese mit dem teilchenförmigen SiO₂ bei 35 bis 45° C mit weniger als 200 U/Min für 40 bis 100 Minuten zu mischen, und es ist entsprechend Wasser hinzuzusetzen je nach gewünschter Konzentration und danach zu homogenisieren.

Bei der Aufbereitung von z.B. TiO₂ ist es zweckmäßig, ölige Dispersionen mit Viskositäten von 13500-200000 mPa·s (cp) einzusetzen und entsprechende Mahlvorgänge dem Einmischen des SiO₂ voranzustellen.

Bei der Aufbereitung von z.B. ZnO ist es zweckmäßig, ölige oder wachshaltige Dispersionen mit Viskositäten von 20000-50000 cps einzusetzen und entsprechende Mahlvorgänge dem Einmischen des ZnO voranzustellen.

Es können Gemische einzelner anderer anorganischer Stoffe mit dem sphärischen SiO₂ hergestellt und dann in die kosmetische Formulierung nacheinander eingebracht werden. Es können aber auch gleich fertige Gemische von mehreren anderen anorganischen Stoffen, z.B. TiO₂/ZnO/SiO₂ hergestellt und dann der kosmetischen Formulierung zugesetzt werden.

Dabei ist für die Agglomeratbildung zu beachten, daß der anfängliche Rührvorgang beim Einbringen des SiO₂ z.B in eine TiO₂-Dispersion sehr langsam erfolgt, z.B. mit 300-400 U/Min und bei Temperaturen zwischen 35 und 42 °C, dann homogenisiert wird, und schließlich die zweite Dispersion (z.B. ZnO) ebenfalls unter langsamem Rühren und bei Temperaturen von 40 bis 48 °C eingetragen wird, und erst dann nochmals homogenisiert wird.

Geeignete Homogenisierungsbedingungen sind z.B. 3000 bis 5000 U/Min.

Die Einhaltung des pH-Wertes ist über den gesamten Mischvorgang zu beachten und kann z.B. über zugesetzte Pufferlösungen, wie Phosphatpuffer gesteuert werden.

Für den Falle der Herstellung fester kosmetischer Zusammensetzungen, wie Preßpuder, entfällt die Wasserzugabe, und es wird statt dessen dem trockenen Gemisch ein Bindemittel hinzugesetzt, das aus mehreren Komponenten besteht und meist auf ölbasis existiert.

Die erfindungsgemäßen Zusammensetzungen mit z. B. Eisenoxiden lassen sich auch ausgezeichnet für die dekorative Kosmetik nutzen, z. B. für Make up's, Lippenstifte, Tönungscremes usw.

Die Erfindung soll nachstehend durch Beispiele näher erläutert werden. Alle Prozentangaben sind auf das Gewicht (Masse) bezogen, sofern keine anderen Angaben gemacht sind.

### Beispiel 1 Herstellung einer Agglomeratdispersion

Es wurde monodisperses, unporöses, sphärisches SiO₂ (Merck) mit einer Teilchengröße von 0,1 µm im Verhältnis 1:30 mit sphärischem TiO₂ trocken bei 35-36 °C und 140 U/Min für 8 Minuten miteinander vermischt. Danach wurde Wasser hinzugegeben und bei 320 U/Min 30 Minuten bis zum Erhalt einer pastenförmigen Konsistenz gerührt. Zu dem Gemisch wurde dann weiteres Wasser hinzugegeben und 20 Minuten bei 3800 U/Min homogenisiert, so daß man eine Dispersion mit eine Viskosität von etwa 23.000 mPa·s (cp) erhielt. Der pH-Wert lag während dieser zeit bei etwa 6. Die Agglomerate hatten eine mittlere Teilchengröße von 0,95 µm, gemessen mit einen Mastersizer (Malvern Instruments Ltd., Worcs, Großbritannien)

In gleicher Weise wurden unter ähnlichen Bedingungen Dispersionen mit ZnO und Fe₂O₃ hergestellt.

### Beispiel 2 Flüssiges Make-up (SPF15)

| **Phase A** | | |
|---|---|---|
| Oil Carnation | | 19 % |
| Silikonöl | | 10 % |
| Jojobaöl | | 5 % |
| Magnesiumstearate | | 2 % |

| **Phase B** | | |
|---|---|---|
| Wasser | | q.s. ad 100 |
| Magnesium sulfate | | 0,9 % |
| Farbe | | 4 % |
| Kaolin modifiziert mit SiO₂ gemäß WO 96/17588 | | 2,5 % |
| erfindungsgemäßes TiO₂/SiO₂ (Verhältnis 4:1) | | 5,0 % |
| erfindungsgemäßes ZnO/SiO₂ (Verhältnis 1:1) | | 2,0 % |

| **Phase C** | | |
|---|---|---|
| Pholosinkomplex nach DE-Patentanmeldung 19654508.0 | | 2,0 % |
| bestehend aus: | | |
| Laminaria saccharina-Extrakt | 40% | |
| Lilium candidum-Extrakt | 45% | |
| Glycerrhetinsäure | 10% | |
| Matricaria recutita-Extrakt | 5% | |

| **Phase D** | | |
|---|---|---|
| asymmetrische lamellare Aggregate mit 2 % hartmagnetischen Einbereichsteilchen gemäß WO 95/03061 | | 1,0 % |
| Palmöl | | 0,5 % |

Die Bestandteile der Phasen A und B wurden getrennt bei etwa 55°-C verrührt. Dabei wurden die erfindungsgemäßen Aggregatdispersionen mit TiO₂ und ZnO nach der Verfahrensweise von Beispiel 1 mit entsprechenden Ausgangsmengen hergestellt. Anschließend wurden die Phasen A und B zusammengegeben, gut homogenisiert und auf 40° C abgekühlt. Danach erfolgte die Zugabe der Phase C und D. Man erhielt ein wasserresistentes flüssiges Make-up mit sehr gutem Hautgefühl, gleichmäßiger Auftragsfähigkeit und lang anhaltendem Aussehen. Der Lichtschutzfaktor SPF war sehr gut stabilisiert.

### Beispiel 3 O/W-Emulsion (SPF 50)

| **Phase A** | |
|---|---|
| Glyceryl Stearate/PEG 100 Stearate | 4,5 % |
| Cetearyl Alcohol | 2,0 % |
| Isohexadecane | 1,5 % |

| **Phase B** | |
|---|---|
| Wasser | q.s. ad 100 |
| Glycerine | 2,0 % |
| erfindungsgemäßes TiO₂/SiO₂ (Verhältnis 70:30) | 25 % |
| erfindungsgemäßes ZnO/SiO₂ (Verhältnis 3:1) | 5 % |
| Kaolin modifiziert mit SiO₂ gemäß WO 96/17588 | 3,0 % |
| Carbomer | 0,1 % |

| **Phase C** | |
|---|---|
| Triethanolamine | 0,1 % |

| **Phase D** | |
|---|---|
| Siliconöl | 2,0 % |
| Babassuöl | 1,5 % |
| Konservierungsmittel | 0,5 % |

| **Phase E** | |
|---|---|
| Bäckerhefe-Aufschlußprodukt nach WO 94/13783 mit Sauerstoff bis zur Sättigung beladene asymmetrische lamellare Aggregate aus Phospholipiden mit 30% Phosphatidylcholin und Perfluordecalin | 0,5 % |
| gemäß WO 94/00109 | 2,5 % |
| Palmöl | 0,5 % |

Die Phasen A und B wurden getrennt durch Vermischen der einzelnen Bestandteile, wobei die Aggregate mit SiO₂ entsprechend Beispiel 1 hergestellt worden waren, bei etwa 55° C zubereitet. Anschließend wurden beide Phasen vermischt und homogenisiert. Nach Abkühlen auf etwa 40° C erfolgte die Zugabe der Phase C und der ebenfalls separat hergestellten Phasen D und E. Die erhaltene, gut homogenisierte O/W-Emulsion hatte einen sehr beständigen und hohen Lichtschutzfaktor, ließ sich ausgezeichnet und homogen auf der Haut verteilen und war sehr beständig.

### Beispiel 4 Sonnenschutzpuder mit UVA/UVB-Schutz (SPF 20)

| | |
|---|---|
| Kaolin modifiziert mit SiO₂ nach WO 96/17588 | 10 % |
| normales Kaolin | 5 % |
| Talcum | q.s. ad 100 |
| Farbe | 4,0 % |
| erfindungsgemäßes TiO₂/SiO₂ (Verhältnis 50:50) | 7 % |
| erfindungsgemäßes ZnO/SiO₂ (Verhältnis 70:30) | 3 % |
| erfindungsgemäßes Fe₂O₃/SiO₂ (Verhältnis 60:40) | 3 % |

Die jeweils getrennt voneinander mit SiO₂ vermischten anderen anorganischen sphärischen Teilchen TiO₂, ZnO und Fe₂O₃ wurden bei Raumtemperatur mit dem Talcum, dem Kaolin und der Farbe vermischt. Danach erfolgte die Zugabe eines Bindemittels für den Preßpuder. Das Bindemittel bestand aus Jojobaöl, Babassuöl und Siliconöl.

## Patentansprüche

1. Kosmetische Zusammensetzungen mit agglomerierten Substraten, **gekennzeichnet durch** einen Gehalt an sphärischen, unporösen SiO₂-Teilchen, die eine Teilchengröße im Bereich von 0,05 bis 1,5 µm haben, und einen Gehalt an anderen anorganischen teilchenförmigen Stoffen mit sphärischer Struktur, wobei das sphärische SiO₂ mit den anderen anorganischen Stoffen definierte Agglomerate mit einer Teilchengröße im Bereich von 0,06 µm bis 5 µm bildet und wobei der Anteil der Agglomerate an der kosmetischen Zusammensetzung im Bereich von 0,1 bis 30 Gew-% liegt,
erhältlich **durch** Vermischen der sphärischen, unporösen SiO₂-Teilchen und der anderen anorganischen teilchenförmigen Stoffe mit sphärischer Struktur, die trocken oder als ölige Dispersion mit einer Viskosität von 13500 bis 200000 mPa·s (cp) vorliegen, einzeln oder im Gemisch unter Einhaltung eines pH-Wertes von 5,5 bis 7 in der Weise, daß ein Teil des Gesamtwassers bis zum Erreichen einer pastenförmigen Konsistenz hinzugegeben und 20 bis 100 Minuten gerührt wird, dann das restliche Wasser unter Beibehaltung des pH-Wertes hinzugegeben und bei 3000 bis 5000 U/Min homogenisiert wird, wobei die Temperatur während der Mischvorgänge im Bereich von 35 bis 48 °C liegt, und die erhaltene Dispersion in üblicher Weise mit einem Anteil der
Agglomerate an der kosmetischen Zusammensetzung im Bereich von 0,1 bis 30 Gew-% in die kosmetische Zusammensetzung eingearbeitet wird.

2. Zusammensetzungen nach Anspruch 1, **dadurch gekennzeichnet, daß** die anderen anorganischen teilchenförmigen Stoffe ausgewählt sind unter Pigmenten; Oxiden wie Eisenoxide, Zinkoxid, Titaniumdioxid, Zirkoniumdioxid; hartmagnetischen Bariumhexaferrit-Einkristallen; hartmagnetischen Strontiumhexaferrit-Einkristallen; und Gemischen davon.

3. Zusammensetzungen nach Anspruch 1 oder 2, **dadurch gekennzeichnet, daß** das Verhältnis der anderen anorganischen teilchenförmigen Stoffe zu dem sphärischen SiO₂ im Bereich von 100:1 bis mehr als 1:1, vorzugsweise im Bereich von 20:1 bis 2:1 liegt.

4. Zusammensetzungen nach Anspruch 2, **dadurch gekennzeichnet, daß** die anderen anorganischen teilchenförmigen Stoffe ausgewählt sind unter Zinkoxid und Titaniumdioxid.

5. Zusammensetzungen nach einem der Ansprüche 1 bis 4, **dadurch gekennzeichnet, daß** sie als Creme, Lotion, Sonnenschutzpräparat, Lippenstift, Make-up, Maske, oder Gel vorliegen.

6. Zusammensetzungen nach einem der Ansprüche 1 bis 5, **dadurch gekennzeichnet, daß** in Sonnenschutzzubereitungen mit verstärktem UVA-Schutz das Verhältnis Zinkoxid und Titaniumdioxid im Bereich von 1:1 bis 10:1 liegt.

7. Zusammensetzungen nach einem der Ansprüche 1 bis 5, **dadurch gekennzeichnet, daß** in Sonnenschutzzubereitungen mit verstärktem UVB-Schutz das Verhältnis Zinkoxid und Titaniumdioxid im Bereich von 1:20 bis 1:100 liegt.

8. Zusammensetzungen nach einem der Ansprüche 1 bis 7, **dadurch gekennzeichnet, daß** der Anteil der Agglomerate an der kosmetischen Zusammensetzung im Bereich von 0,5 bis 25 Gew-% liegt.

9. Verfahren zur Herstellung von kosmetischen Zusammensetzungen mit agglomerierten Substraten, **dadurch gekennzeichnet, daß** sphärische, unporöse SiO₂-Teilchen, die eine Teilchengröße im Bereich von 0,05 bis 1,5 µm haben und andere anorganische teilchenförmige Stoffe mit sphärischer Struktur miteinander vermischt werden zum Erhalt von definierten Agglomeraten mit einer Teilchengröße von 0,06 µm bis 5 µm, wobei die anderen anorganischen teilchenförmigen Stoffe mit sphärischer Struktur, die trocken oder als ölige Dispersion mit einer Viskosität von 13500 bis 200000 mPa·s (cp) vorliegen, einzeln oder im Gemisch unter Einhaltung eines pH-Wertes von 5,5 bis 7 in der Weise mit den sphärischen, unporösen SiO₂-Teilchen vermischt werden, daß ein Teil des Gesamtwassers bis zum Erreichen einer pastenförmigen Konsistenz hinzugegeben und 20 bis 100 Minuten gerührt wird, dann das restliche Wasser unter Beibehaltung des pH-Wertes hinzugegeben und für 20 bis 60 Minuten homogenisiert wird, wobei die Temperatur während der Mischvorgänge im Bereich von 35 bis 48 °C liegt, und die erhaltene Dispersion in üblicher Weise mit einem Anteil der Agglomerate an der kosmetischen Zusammensetzung im Bereich von 0,1 bis 30 Gew-% in die kosmetische Zusammensetzung eingearbeitet wird.

10. Verfahren nach Anspruch 9, **dadurch gekennzeichnet, daß** bei trockenen kosmetischen Zusammensetzungen anstelle des Wassers ein ölhaltiges Bindemittel zugesetzt wird.

11. Verfahren nach Anspruch 9, **dadurch gekennzeichnet, daß** das Rühren bei 300 bis 400 U/Min erfolgt.

12. Verfahren nach Anspruch 9, **dadurch gekennzeichnet, daß** das Homogenisieren bei 3000 bis 5000 U/Min erfolgt.

## Claims

1. Cosmetic compositions containing agglomerated substrates, comprising a content of spherical, non-porous SiO₂ particles with a particle size ranging from 0.05 to 1.5 µm, and a content of other inorganic particle-shaped materials of spherical structure; with the spherical SiO₂ forming defined agglomerates with the other inorganic materials, of a particle size ranging from 0.06 µm to 5 µm and with the proportion of agglomerates in the cosmetic composition ranging from 0.1 to 30 %wt;
available by mixing the spherical non-porous SiO₂ particles with the other inorganic particle-shaped materials of spherical structure present dry or as an oily dispersion with a viscosity of 13,500 to 200,000 Pa·s (cps), individually or mixed, maintaining a pH value of 5.5 to 7 such that part of the total water is to be added until a paste-like consistency is attained, with stirring for 20 to 100 minutes, with the remaining water subsequently being added while the pH value is maintained and homogenisation taking place at 3,000 to 5,000 rpm; with the temperature during the mixing processes ranging from 35 to 48 °C, and with the dispersion obtained being worked into the cosmetic composition in the usual manner with the proportion of agglomerates in the cosmetic composition being between 0.1 and 30 %wt.

2. A compositions according to claim 1 wherein the other inorganic particle-shaped materials are selected from among pigments; oxides such as iron oxides, zinc oxide, titanium dioxide, zirconium dioxide; magnetically hard barium hexaferrite single-crystals; magnetically hard strontium hexaferrite single-crystals; and mixtures thereof.

3. A compositions according to claim 1 or 2, wherein the proportion of the other inorganic particle-shaped materials to the spherical SiO₂ ranges from 100:1 to more than 1:1, preferably from 20:1 to 2:1.

4. A compositions according to claim 2 wherein the other inorganic particle-shaped materials are selected from among zinc oxide and titanium dioxide.

5. A compositions according to one of claims 1 to 4 wherein they are a creme, lotion, sunscreen preparation, lipstick, make-up, mask or gel.

6. A compositions according to one of claims 1 to 5, wherein in sunscreen preparations with improved UV-A protection the proportion of zinc oxide to titanium dioxide ranges from 1:1 to 10:1.

7. A compositions according to one of claims 1 to 5, wherein in sunscreen preparations with improved UV-B protection the proportion of zinc oxide to titanium dioxide ranges from 1:20 to 1:100.

8. A compositions according to one of claims 1 to 7 wherein the proportion of agglomerates in the cosmetic composition ranges from 0.5 to 25 %wt.

9. A process for producing cosmetic compositions with agglomerated substrates, comprising intermixing of spherical non-porous SiO₂ particles with a particle size ranging from 0.05 to 1.5 µm, with other inorganic particle-shaped materials of spherical structure, to obtain defined agglomerates of a particle size of 0.06 µm to 5 µm, with the other inorganic particle-shaped materials with spherical structure which are present dry or as an oily dispersion with a viscosity of 13,500 to 200,000 Pa·s (cps) being mixed together individually or as a mixture with the spherical non-porous SiO₂ particles while maintaining a pH value of 5.5 to 7 such that part of the total water is to be added until a paste-like consistency is attained, with stirring for 20 to 100 minutes, with the remaining water subsequently being added while the pH value is maintained and homogenisation taking place for 20 to 60 minutes; with the temperature during the mixing processes ranging from 35 to 48 °C, and with the dispersion obtained being worked into the cosmetic composition in the usual manner with the proportion of agglomerates in the cosmetic composition being between 0.1 and 30 %wt.

10. A process according to claim 9 wherein in dry cosmetic composition an oily binder is added instead of water.

11. A process according to claim 9, wherein stirring takes place at 300 to 400 rpm.

12. A process according to claim 9, wherein homogenisation takes place at 3,000 to 5,000 rpm.

## Revendications

1. Compositions cosmétiques à substrats agglomérés **caractérisées par** une teneur en particules de SiO₂ sphérique non poreux dont la granulométrie est comprise entre 0,05 et 1,5 µm, et une teneur en d'autres substances inorganiques sous forme de particules de structure sphérique, le SiO₂ sphérique formant avec les autres substances inorganiques des agglomérats définis dont la granulométrie est comprise entre 0,06 et 5 µm et la proportion des agglomérats dans la composition cosmétique se situant entre 0,1 et 30% en poids,
obtenues en mélangeant les particules de SiO₂ sphérique non poreuses et les autres substances organiques sous forme de particules de structure sphérique se présentant sèches ou en dispersion dans l'huile de viscosité comprise entre 13500 et 200000 mPa.s (cp), séparément ou en mélange, en maintenant un pH de 5,5 à 7 de sorte que une partie de l'eau totale soit ajoutée jusqu'à obtention d'une consistance pâteuse et agitée pendant 20 à 100 minutes et que le reste de l'eau soit introduit en contrôlant le pH et homogénéisé à 3000-5000 t/min, la température étant comprise entre 35 et 48°C pendant le mélange et la dispersion obtenue étant habituellement introduite dans la composition cosmétique de façon à ce que la proportion des agglomérats dans cette dernière se situe entre 0,1 et 30% en poids.

2. Compositions selon la revendication 1 **caractérisées en ce que** les autres substances inorganiques sous forme de particules sont choisies parmi des pigments ; des oxydes tels que l'oxyde de fer, l'oxyde de zinc, le dioxyde de titane, le dioxyde de zirconium ; des monocristaux magnétiques durs d'hexaferrite de baryum ; ; des monocristaux magnétiques durs d'hexaferrite de strontium ; et des mélanges de ces derniers.

3. Compositions selon la revendication 1 ou 2 **caractérisées en ce que** le rapport des autres substances inorganiques sous forme de particules par rapport au SiO₂ sphérique se situe dans une plage allant de 100:1 à plus de 1:1, de préférence dans une plage allant de 20:1 à 2:1.

4. Compositions selon la revendication 2 **caractérisées en ce que** les autres substances inorganiques sous forme de particules sont choisies parmi l'oxyde de zinc et le dioxyde de titane.

5. Compositions selon les revendications 1 à 4 **caractérisées en ce qu'**elles se présentent sous forme de crème, lotion, préparation de protection solaire, bâton à lèvres, maquillage, masque ou gel.

6. Compositions selon les revendications 1 à 5 **caractérisées en ce que** le rapport oxyde de zinc/ dioxyde de titane se situe dans la plage 1:1 à 10:1 dans les préparations de protection solaire à protection renforcée contre les UVA.

7. Compositions selon l'une des revendications 1 à 5 **caractérisées en ce que** le rapport oxyde de zinc/ dioxyde de titane se situe dans la plage 1:20 à 1:100 dans les préparations de protection solaire à protection renforcée contre les UVB.

8. Compositions selon l'une des revendications 1 à 7 **caractérisées en ce que** la proportion des agglomérats dans la composition cosmétique est comprise entre 0,5 et 25% en poids.

9. Procédé de fabrication de compositions cosmétiques à substrats agglomérés **caractérisé en ce que** des particules sphériques non poreuses de SiO₂ de granulométrie comprise entre 0,05 et 1,5 µm et d'autres substances inorganiques sous forme de particules, de structure sphérique, sont mélangées les unes avec les autres pour obtenir des agglomérats définis de granulométrie comprise entre 0,06 et 5 µm, les autres substances inorganiques sous forme de particules, de structure sphérique se présentant sèches ou en dispersion dans l'huile d'une viscosité comprise entre 13500 et 200000 mPa.s (cp), séparément ou en mélange, et étant mélangées aux particules sphériques non poreuses de SiO₂ à un pH maintenu entre 5,5 et 7 de façon à ce qu'une partie de l'eau totale soit ajoutée jusqu'à obtention d'une consistance pâteuse et agitée pendant 20 à 100 minutes et à ce que l'eau restante soit ensuite ajoutée sous contrôle du pH et homogénéisé pendant 20 à 60 minutes, la température pendant les processus de mélange étant comprise entre 35 et 48°C et la dispersion obtenue étant généralement introduite dans la composition cosmétique de façon à ce que la proportion des agglomérats dans cette dernière se situe dans la plage 0,1 à 30% en poids.

10. Procédé selon la revendication 9 **caractérisé en ce qu'**un liant huileux est ajouté à la place de l'eau aux compositions cosmétiques sèches.

11. Procédé selon la revendication 9 **caractérisé en ce que** l'agitation s'effectue à 300-400 t/min.

12. Procédé selon la revendication 9 **caractérisé en ce que** l'homogénéisation s'effectue à 3000/5000 t/min.
